(19) Europäisches Patentamt
European Patent Office
Office européen des brevets

(11) Veröffentlichungsnummer: **0 091 639**
**B1**

(12) # EUROPÄISCHE PATENTSCHRIFT

(45) Veröffentlichungstag der Patentschrift: **16.06.87**

(21) Anmeldenummer: **83103304.8**

(22) Anmeldetag: **05.04.83**

(51) Int. Cl.⁴: **C 07 C127/19**, C 07 C125/065, C 07 C149/437, C 07 C155/02, C 07 C103/38, C 07 C103/44, C 07 C103/737, C 07 C149/42, C 07 D295/20, A 01 N 37/22, A 01 N 43/34

(54) **Anilinderivate, Verfahren zu ihrer Herstellung und ihre Verwendung zur Bekämpfung unerwünschten Pflanzenwuchses.**

(30) Priorität: **10.04.82 DE 3213376**

(43) Veröffentlichungstag der Anmeldung: **19.10.83 Patentblatt 83/42**

(45) Bekanntmachung des Hinweises auf die Patenterteilung: **16.06.87 Patentblatt 87/25**

(84) Benannte Vertragsstaaten: **BE DE FR GB IT**

(56) Entgegenhaltungen: **DE-B- 1 204 880**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft
Carl-Bosch-Strasse 38
D-6700 Ludwigshafen (DE)**

(72) Erfinder: **Koenig, Karl-Heinz, Dr.
Pierstrasse 8 a
D-6710 Frankenthal (DE)**
Erfinder: **Schirmer, Ulrich, Dr.
Berghalde 79
D-6900 Heidelberg (DE)**
Erfinder: **Liesner, Michael, Dr.
Haardtstrasse 6
D-6714 Weisenheim (DE)**
Erfinder: **Plath, Peter, Dr.
Berner Weg 24
D-6700 Ludwigshafen (DE)**
Erfinder: **Wuerzer, Bruno, Dr. Dipl.-Landwirt
Ruedigerstrasse 13
D-6701 Otterstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 091 639 B1

**Beschreibung**

Die Erfindung betrifft Anilinderivate, Verfahren zu ihrer Herstellung, Herbizide, die diese Verbindungen als Wirkstoffe enthalten, sowie ein Verfahren zur Bekämpfung unerwünschten Pflanzenwuchses mit diesen Verbindungen.

Es ist bekannt, daß N-(Trichlorbenzyl-thiomethyl-phenyl)-harnstoffe und N-(3-Phenoxymethyl-phenyl)-amide herbizid wirksam sind (DE-PS 12 04 880 ; DE-OS 28 55 699).

Es wurde gefunden, daß Anilinderivate der Formel

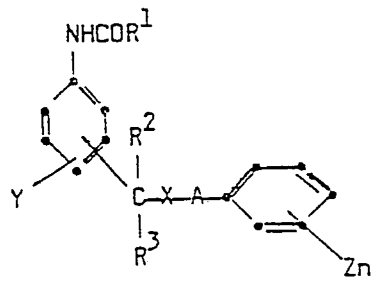

(I)

in der die Reste —NH—COR[1] und

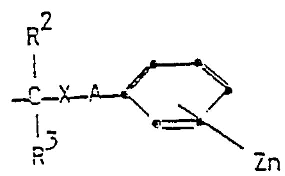

in m- oder p-Stellung stehen und

$R^1$ Alkoxy, Alkenoxy, Alkinoxy, Alkylthio oder gegebenenfalls durch Halogen oder Alkoxy mit 1 bis 5 Kohlenstoffatomen substituiertes Alkyl oder Alkenyl mit jeweils bis zu 4 Kohlenstoffatomen oder Cycloalkyl mit 3 bis 6 Kohlenstoffatomen oder den Rest $—NR^4R^5$, wobei $R^4$ und $R^5$ unabhängig voneinander Wasserstoff, Alkyl, Alkenyl, Alkinyl oder Alkoxy mit jeweils bis zu 4 Kohlenstoffatomen oder Cycloalkyl oder Cycloalkoxy mit 3 bis 6 Kohlenstoffatomen bedeuten oder $R^4$ und $R^5$ zusammen eine gegebenenfalls durch Methyl substituierte und gegebenenfalls Sauerstoff als Kettenglied enthaltende Alkylenkette mit bis zu 6 Kohlenstoffatomen bedeuten,

$R^2$ und $R^3$ unabhängig voneinander Wasserstoff oder Alkyl mit 1 bis 3 Kohlenstoffatomen,

X Sauerstoff oder den Rest $—NR^6—$, wobei $R^6$ ein Alkyl mit 1 bis 5 Kohlenstoffatomen bedeutet,

Y Wasserstoff, Halogen, Methyl, Methoxy oder Trifluormethyl,

A eine gegebenenfalls durch Alkyl mit 1 bis 5 Kohlenstoffatomen substituierte und gegebenenfalls Sauerstoff als Kettenglied enthaltende Alkylen- oder Alkenylenkette mit bis zu 5 Kohlenstoffatomen,

Z Wasserstoff, Halogen, Alkyl, Alkoxy, Halogenalkyl oder Halogenalkoxy mit jeweils bis zu 6 Kohlenstoffatomen, Phenyl oder gegebenenfalls durch Halogen, Nitro, Alkyl, Halogenalkyl oder Alkoxy mit jeweils 1 bis 3 Kohlenstoffatomen substituiertes Phenoxy und

n die Zahlen 1, 2 oder 3 bedeuten,

eine herbizide Wirkung haben.

Die Substituenten in Formel I können die folgenden Bedeutungen haben :

$R^1$ bedeutet Alkoxy, Alkenoxy, Alkinoxy, Alkylthio oder gegebenenfalls durch Halogen oder Alkoxy mit 1 bis 4 Kohlenstoffatomen substituiertes Alkyl oder Alkenyl mit jeweils bis zu 5 Kohlenstoffatomen, beispielsweise Methoxy, Ethoxy, n-Propoxy, Isopropoxy, sec-Butoxy, tert.-Butoxy, n-Pentoxy, Propargyloxy, Allyloxy, 1-Methyl-propgargyloxy, Methylthio, Ethylthio, n-Propylthio, sec.-Butylthio, tert.-Butylthio, Methyl, Ethyl, n-Propyl, n-Butyl, sec.-Butyl, tert.-Butyl, Isobutyl, n-Pentyl, sec.-Pentyl, Chlormethyl, 1,1-Dichlorethyl, Dichlormethyl, Trichlormethyl, Methoxymethyl, 1-Methoxyethyl, 2-Methoxyethyl, Vinyl, Propenyl, Isopropenyl, Allyl, Cycloalkyl mit 3 bis 6 Kohlenstoffatomen, beispielsweise Cyclopropyl, Cyclopentyl, Cyclohexyl, Cycloalkoxy mit 3 bis 6 Kohlenstoffatomen, beispielsweise Cyclohexoxy, oder den Rest $—NR^4R^5$, wobei $R^4$ und $R^5$ unabhängig voneinander Wasserstoff, Alkyl, Alkenyl, Alkinyl oder Alkoxy mit jeweils bis zu 4 Kohlenstoffatomen, beispielsweise Methyl, Ethyl, Isopropyl, n-Butyl, sec-Butyl, Isobutyl, tert.-Butyl, Allyl, 1,1-Dimethylpropargyl, Methoxy, Ethoxy, oder Cycloalkyl mit 3 bis 6 Kohlenstoffatomen, beispielsweise Cyclopropyl, Cyclopentyl, Cyclohexyl, oder $R^4$ und $R^5$ zusammen eine gegebenenfalls durch Methyl substituierte und gegebenenfalls Sauerstoff als Kettenglied enthaltende Alkylenkette mit bis zu 6 Kohlenstoffatomen, beispielsweise Trimethylen, Tetramethylen, 1,4-Dimethyltetramethylen, Pentamethylen, Hexamethylen, 3-Oxapentamethylen, 1-Oxapentamethylen, 1-Oxa-tetramethylen, bedeuten.

$R^2$ und $R^3$ bedeuten unabhängig voneinander Wasserstoff oder Alkyl mit 1 bis 3 Kohlenstoffatomen, Methyl, Ethyl, n-Propyl, i-Propyl.

X bedeutet Sauerstoff oder den Rest $—NR^6—$, wobei $R^6$ Alkyl mit 1 bis 5 Kohlenstoffatomen, beispielsweise Methyl, Ethyl, Pentyl bedeutet.

Y bedeutet außer Wasserstoff, Methyl, Methoxy, Trifluormethyl oder Halogen, wie Fluor, Chlor, Brom.

A bedeutet eine Alkylen- oder Alkenylenkette mit bis zu 5 Kohlenstoffatomen und gegebenenfalls mit Sauerstoff als Kettenglied, die durch Alkyl mit 1 bis 5 Kohlenstoffatomen, insbesondere durch Methyl, substituiert sein kann, beispielsweise $—CH_2—$, $—CH(CH_3)—$, $—(CH_2)_3—$, $—(CH_2)_2—$, $—CH_2CH(CH_3)—$, $—(CH_2)_5—$, $—CH_2—CH{=}CH—$, $—CH_2—CH_2—O—$, $—CH_2—CH(CH_3)O—$, $—(CH_2)_4O—$, $—CH_2CH_2OCH_2—$ oder $—CH_2CH_2CH_2OCH_2—$.

Z steht für Wasserstoff, Halogen, beispielsweise Fluor, Chlor, Brom, Alkyl, Alkoxy, Halogenalkyl oder Halogenalkoxy mit jeweils bis zu 6 Kohlenstoffatomen, beispielsweise Methyl, Ethyl, Isopropyl, tert.-Butyl, n-Hexyl, Methoxy, Ethoxy, Isopropoxy, tert.-Butoxy, n-Hexoxy, Trifluormethyl, 1,1,2-Trifluor-2-chlorethoxy, Difluormethoxy, Phenyl, Phenoxy, 2,4-Dichlorphenoxy, 2-Chlor-4-trifluormethylphenoxy oder 3-Chlor-4-trifluormethylphenoxy, Z steht in ortho-, meta- oder para-Stellung zu A, n ist vorzugsweise 1, kann jedoch auch 2 oder 3 bedeuten, insbesondere, wenn Z für Fluor, Chlor, Brom oder Methyl steht.

Bevorzugte Verbindungen der Formel I sind solche, bei denen $R^1$ den Rest $—N(OCH_3)(CH_3)$, Y Wasserstoff oder Halogen, insbesondere Chlor, A Methylen oder Dimethylen, $R^2$ Wasserstoff oder Methyl, $R^3$ Wasserstoff, X Sauerstoff, Z Halogen, insbesondere Chlor, oder Alkyl mit bis zu 4 Kohlenstoffatomen, insbesondere Methyl oder Methoxy, und n 0 oder 1 bedeuten.

Man erhält die Anilinderivate der Formel I durch Umsetzung von Aminen der Formel

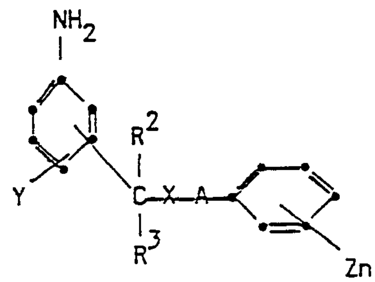

(II)

in der $R^2$, $R^3$, X, Y, A, Z und n die obengenannten Bedeutungen haben, mit Verbindungen der Formel

$$R^1—CO—X \qquad (III),$$

in der $R^1$ die obengenannten Bedeutungen hat und X eine Abgangsgruppe, vorzugsweise Halogen, wie Chlor oder Brom, oder $R^1—CO—O—$ bedeutet.

Die Umsetzung wird in einem inerten organischen Lösungsmittel durchgeführt. Geeignet sind Ether, wie Tetrahydrofuran, Dimethoxyethan, Diethylether, Methyl-tert.-butylether, Ester wie Essigsäureethylester, gegebenenfalls chlorierte aliphatische oder aromatische Kohlenwasserstoffe, wie Toluol, Dichlormethan, Pyridin. Auch Gemische dieser Lösungsmittel können verwendet werden. Die Menge an Lösungsmittel, bezogen auf Amin der Formel II beträgt 100 bis 5 000 Gew.%.

Zweckmäßigerweise wird die Umsetzung in Gegenwart eines Säureakzeptors durchgeführt. In Betracht kommen Alkalihydroxide, Alkalicarbonate, Alkalihydrogencarbonate, Erdalkalihydroxide, Erdalkalicarbonate, Erdalkalihydrogencarbonate, Erdalkalioxide oder Amine, beispielsweise Natriumhydrogencarbonat, Kaliumcarbonat, Triethylamin, Pyridin, N,N-Dimethylanilin, N,N-Dimethyl-N-cyclohexylamin, Chinolin. Die Menge an Säureakzeptor beträgt 1 bis 4 Mol, bezogen auf 1 Mol Verbindung der Formel III.

Die Ausgangsstoffe der Formeln II und III werden bevorzugt in äquimolarem Verhältnis miteinander umgesetzt. Die Reaktionstemperatur liegt zwischen 20 und 80 °C, vorzugsweise zwischen 20 und 30 °C.

Die Anilinderivate der Formel I werden vorzugsweise durch Umsetzung von Verbindungen der Formel

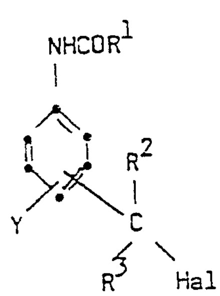

(IV)

in der Hal für Chlor oder Brom steht und $R^1$, $R^2$, $R^3$ und Y die oben genannten Bedeutungen haben, mit einer Verbindung der Formel

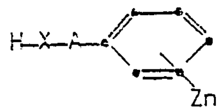

(V)

in der X, A, Z und n die oben genannten Bedeutungen haben, erhalten.

Die Umsetzung wird entweder in einem inerten Lösungsmittel oder in einem Überschuß von V durchgeführt. Als Lösungsmittel kommen Ether, wie Diethylether, Methyl-tert.-butylether, Tetrahydrofuran, Ester, wie Essigsäureethylester, gegebenenfalls chlorierte aliphatische oder aromatische Kohlenwasserstoffe, wie Ligroin, Toluol, Cyclohexan, Benzin, Dichlormethan, Chloroform, Chlorbenzol, o-, m-, p-Dichlorbenzol, Nitrobenzol, Ketone, wie Aceton, Nitrile, wie Acetonitril, Dimethylsulfoxid oder Dimethylformamid in Betracht. Auch Gemische dieser Lösungsmittel können verwendet werden.

Zweckmäßigerweise werden die Verbindungen V in Form ihrer Alkalisalze eingesetzt, man kann aber auch mit Säureakzeptoren wie Alkalihydroxid, Alkalicarbonat, Alkali-hydrogencarbonat, tert.-Aminen, wie Triethylamin, Dimethylcyclohexylamin, N,N-Dimethylanilin oder Pyridin arbeiten. Für den Fall, daß in der Verbindung der Formel V X die Bedeutung —$NR^6$— hat, bietet es sich an, die Verbindung V gleichzeitig als Säureakzeptor einzusetzen.

Die Menge an Säureakzeptor beträgt 1 bis 4 Mol, bezogen auf 1 Mol Verbindung der Formel IV.

Die Ausgangsstoffe der Formeln IV und V werden in einem molaren Verhältnis von 1 : 1 bis 1 : 50 miteinander umgesetzt. Die Reaktionstemperatur liegt zwischen 0 und 100 °C, vorzugsweise zwischen 10 und 30 °C.

Die Ausgangsstoffe der Formel II erhält man durch Reduktion entsprechender Nitroverbindungen (Houben-Weyl, Methoden der Org. Chemie, Bd. 4/1c, Seite 506-532, Georg Thieme Verlag, Stuttgart, 1980).

Die Ausgangsstoffe der Formel IV erhält man durch Reaktion entsprechender Isocyanate mit Aminen, Alkoholen oder Mercaptanen (DE-AS 12 04 880 und Annalen der Chemie 562, 75 ff. (1949) bzw. durch Umsetzung entsprechender Hydroxyalkyl-anilinderivate (DE-OS 30 24 538) mit Phosphortrihalogeniden.

Die folgenden Beispiele, die die Herstellung der Anilinderivate der Formel I erläutern, wurden durchgeführt :

## Beispiel 1

42,35 g Methylbenzylamin, 260 ml Ethanol und 30 g 4-Nitrobenzylchlorid wurden langsam zum Sieden erhitzt und 3 Stunden unter Rückfluß gerührt. Nach dem Einengen wurde der Rückstand mit 300 ml Essigester versetzt, 2x mit Wasser gewaschen, über $MgSO_4$ getrocknet, filtriert und eingeengt. Das erhaltene Öl wurde in 750 ml Tetrahydrofuran gelöst und in Gegenwart von 5 g Pd/Tierkohle (5 %ig) bei 25 °C und 1,1 bar Wasserstoffdruck hydriert. Nach 11,5 h $H_2$-Aufnahme wurde abfiltriert und eingeengt. Es wurden 21 g 4-[(N-Benzyl-N-methyl-amino)-methyl]-anilin in Form eines Öls vom $n_D^{25} = 1 \cdot 582\,9$ erhalten.

13,5 g des so erhaltenen Amins wurden in 150 ml Tetrahydrofuran gelöst, mit 10,0 g Natriumhydrogencarbonat versetzt und unter gutem Rühren bei 20 bis 25 °C mit 7,4 g N-Methoxy-N-methylcarbaminsäurechlorid zur Reaktion gebracht. Nach 16-stündigem Nachrühren wurde filtriert, eingeengt, in Essigester gelöst, mit Wasser gewaschen, über $Na_2SO_4$ getrocknet und eingeengt. Es wurden 11 g eines Öls vom $n_D^{25} = 1 \cdot 565\,1$ erhalten, das folgender Strukturformel entspricht :

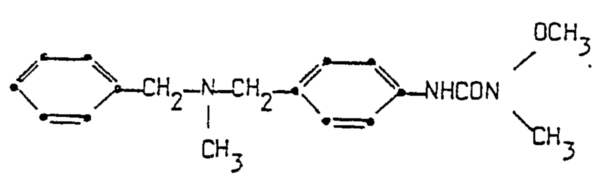

## Beispiel 2

8 Gewichtsteile N-Methoxy-N-methylamin in 50 Gewichtsteilen Toluol werden bei 0 °C mit 23,5 Gewichtsteilen 4-(1-Chlorethyl)-phenylisocyanat in 100 Gewichtsteilen Toluol 30 Minuten lang zur Reaktion gebracht. Der entstandene Niederschlag wurde abgesaugt ; es wurden 12 Gewichtsteile N-[4-(1-Chlorethyl)-phenyl]-N'-methoxy-N'-methylharnstoff vom Schmelzpunkt 86 °C erhalten.

Zu 17 g N-Benzyl-N-methylamin in 50 ml Toluol wurden bei 20 bis 30 °C 17 g N-(4-[1-Chlorethyl] phenyl)-N'-methyl-N'-methoxyharnstoff in 200 ml Toluol zugetropft. Dann wurde 2 Stunden bei 30 °C, dann über Nacht bei Raumtemperatur gerührt. Nach dem Trocknen wurde eingeengt, mit Diethylether verrührt, abgesaugt und getrocknet. Nach dem Umkristallisieren aus Essigester wurden 10,5 g weiße

Kristalle vom Schmelzpunkt 93-94 °C und folgender Strukturformel erhalten :

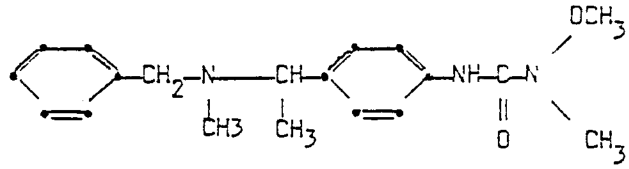

Beispiel 3

66 Gewichtsteile p-(1-Chlorethyl)-phenylisocyanat in 200 Gewichtsteilen Essigester wurden bei 15 °C mit 15 Gewichtsteilen Dimethylamin in 100 Teilen Essigester versetzt und 1 Stunde lang zur Reaktion gebracht. Der entstandene Nierderschlag wurde abgesaugt ; es wurden 77 Gewichtsteile N-[4-(1-Chlorethyl)-phenyl]-N',N'-dimethylharnstoff vom Schmelzpunkt 120 °C erhalten.

Zu 32 g Benzylalkohol wurden langsam 2,3 g 80 %iges Natriumhydrid gegeben, dann wurde 2 Stunden bei 40 bis 45 °C gerührt, und anschließend wurden bei 20 bis 25 °C 15 g N-[4-(1-Chlorethyl)phenyl]-N',N'-dimethylharnstoff in 200 ml Benzylalkohol zugetropft. Nach Rühren über Nacht bei Raumtemperatur wurde der überschüssige Benzylalkohol im Hochvakuum am Rotationsverdampfer abdestilliert und der Rückstand mit Diisopropylether ausgekocht. Nach dem Abkühlen wurde der ausgefallene Feststoff abgesaugt und getrocknet. Es wurden 8 g einer weißen, kristallinen Substanz vom Schmelzpunkt 98 bis 100 °C und folgender Strukturformel erhalten :

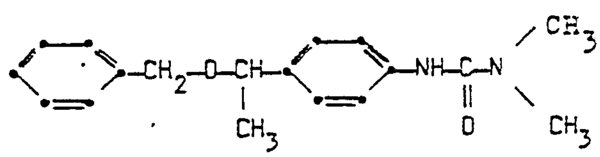

Entsprechend können beispielsweise folgende Anilinderivate der Formel I hergestellt werden :

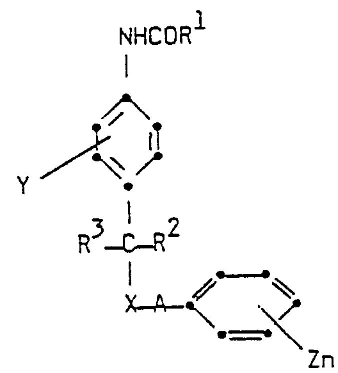

(Siehe Formel Seite 6ff.)

| Nr. | $R^1$ | Y | $R^2$ | $R^3$ | X | A | Zn | Fp. [°C]/$n_D$ |
|---|---|---|---|---|---|---|---|---|
| 6 | $N(CH_3)_2$ | H | H | H | O | $-CH_2-$ | H | 106-108 |
| 7 | $N(CH_3)_2$ | H | H | H | $-N(CH_3)-$ | $-CH_2-$ | H | 112-113 |
| 8 | $N(CH_3)_2$ | H | $CH_3$ | H | $-N(CH_3)-$ | $-CH_2-$ | H | 128-130 |
| 9 | $N(OCH_3)(CH_3)$ | H | H | H | O | $-CH_2-$ | H | $n_D^{20}$ = 1.5769 |
| 10 | $NH(C_2H_5)$ | 3-Cl | H | H | O | $-CH_2-$ | 3-Cl | |
| 11 | $NH(CH_2CH_2CN)$ | 3-Cl | $CH_3$ | H | O | $-CH_2-$ | 4-$CH_3$ | |
| 12 | $N(CH_3)_2$ | H | $CH_3$ | $CH_3$ | O | $-CH_2-$ | H | |
| 13 | $N(CH_3)_2$ | H | $C_2H_5$ | H | O | $-CH_2-$ | H | |
| 14 | $N(CH_3)_2$ | H | H | H | O | $-CH_2-$ | 4-$CF_3$ | |
| 15 | $N(CH_3)_2$ | H | H | H | O | $-CH_2-$ | 4-$CH_3$ | 104-106 |
| 16 | $N(CH_3)_2$ | H | H | H | O | $-CH_2-$ | 4-Cl | 130-132 |
| 17 | $N(CH_3)_2$ | H | H | $C_2H_5$ | O | $-CH_2-$ | 4-$CH_3$ | |
| 18 | $N(CH_3)_2$ | H | H | H | O | $-CH_2-$ | 2,4-$Cl_2$ | 141-145 |
| 19 | $N(CH_3)_2$ | H | H | H | O | $-CH_2-$ | 2,3,6-$Cl_3$ | |
| 20 | $N(CH_3)_2$ | H | H | H | O | $-CH_2-$ | 2,6-$Cl_2$ | |
| 21 | $N(CH_3)_2$ | 3-Cl | H | H | O | $-C(CH_3)_2-$ | H | |
| 22 | $N(CH_3)_2$ | 3-Cl | H | H | O | $-CH_2\underset{CH_3}{C}HCH_2\underset{CH_3}{C}HCH_2-$ | H | |
| 23 | $N(CH_3)_2$ | 3-Cl | $CH_3$ | H | O | $-CH(CH_3)-$ | H | |
| 24 | $N(OCH_3)(CH_3)$ | H | $CH_3$ | H | O | $-CH_2-$ | 4-$CH_3$ | 62-64 |
| 25 | $N(OCH_3)(CH_3)$ | H | H | H | O | $-CH_2-$ | 4-$CH_3$ | 65-70 |

| Nr. | $R^1$ | Y | $R^2$ | $R^3$ | X | A | Zn | Fp. [°C]/$n_D$ |
|---|---|---|---|---|---|---|---|---|
| 26 | $N(OCH_3)(CH_3)$ | H | $CH_3$ | $CH_3$ | O | $-CH_2-$ | H | |
| 27 | $N(OCH_3)(CH_3)$ | H | H | H | O | $-CH-$<br>$CH_3$ | H | Sirup |
| 28 | $N(OCH_3)(CH_3)$ | H | $C_2H_5$ | H | O | $-CH_2-$ | H | |
| 29 | $N(OCH_3)(CH_3)$ | H | $n-C_3H_7$ | H | O | $-CH_2-$ | H | |
| 30 | $N(OCH_3)(CH_3)$ | H | $CH_3$ | H | O | $-CH-$<br>$CH_3$ | H | $n_D^{20} = 1.5545$ |
| 31 | $N(OCH_3)(CH_3)$ | H | $CH_3$ | H | O | $-CH_2-$ | H | 71-72 |
| 32 | $N(OCH_3)(CH_3)$ | H | H | H | O | $-CH-$<br>$CH_3$ | $4-CH_3$ | 73-75 |
| 35 | $N(OCH_3)(CH_3)$ | H | H | H | O | $-CH_2-CH_2-$ | H | 61-63 |
| 36 | $N(OCH_3)(CH_3)$ | H | H | H | O | $-(CH_2)_3-$ | H | |
| 37 | $N(OCH_3)(CH_3)$ | H | H | H | O | $-(CH_2)_4-$ | H | |
| 38 | $N(OCH_3)(CH_3)$ | H | H | H | O | $-(CH_2)_5-$ | H | |
| 39 | $N(OCH_3)(CH_3)$ | H | H | H | O | $-CH_2-CH=CH-$ | H | $n_D^{20} = 1.5906$ |
| 40 | $N(OCH_3)(CH_3)$ | 3-Cl | H | H | O | $-CH_2-$ | H | $n_D^{20} = 1.5765$ |
| 41 | $N(OCH_3)(CH_3)$ | 3-Cl | $CH_3$ | H | O | $-CH_2-$ | H | $n_D^{20} = 1.5682$ |
| 42 | $N(OCH_3)(CH_3)$ | $3-CH_3$ | H | H | O | $-CH_2-$ | H | |
| 43 | $N(OCH_3)(CH_3)$ | $3-OCH_3$ | H | H | O | $-CH_2-$ | H | |

| Nr. | $R^1$ | Y | $R^2$ | $R^3$ | X | A | Zn | Fp. [°C]/$n_D$ |
|---|---|---|---|---|---|---|---|---|
| 44 | $N(OCH_3)(CH_3)$ | 3-$CF_3$ | H | H | O | $-CH_2-$ | H | |
| 45 | $N(OCH_3)(CH_3)$ | 3-Br | H | H | O | $-CH_2-$ | H | |
| 46 | $N(OCH_3)(CH_3)$ | H | H | H | O | $-CH_2-$ | 4-Cl | 44-50 |
| 47 | $N(OCH_3)(CH_3)$ | H | H | H | O | $-CH_2-$ | 4-$OCH_3$ | |
| 48 | $N(OCH_3)(CH_3)$ | H | H | H | O | $-CH_2-$ | 3-$CH_3$ | |
| 49 | $N(OCH_3)(CH_3)$ | H | H | H | O | $-CH_2-$ | 3-Cl | |
| 50 | $N(OCH_3)(CH_3)$ | H | H | H | O | $-CH_2-$ | 2-$CH_3$ | |
| 51 | $N(OCH_3)(CH_3)$ | H | H | H | O | $-CH_2-$ | 2,5-$(CH_3)_2$ | |
| 52 | $N(OCH_3)(CH_3)$ | H | H | H | O | $-CH_2-$ | 4-t-$C_4H_9$ | 58-60 |
| 53 | $N(OCH_3)(CH_3)$ | H | H | H | O | $-CH_2-$ | 4-O-t-$C_4H_9$ | |
| 54 | $N(OCH_3)(CH_3)$ | H | H | H | O | $-CH_2-$ | 3-Phenoxy | |
| 55 | $N(OCH_3)(CH_3)$ | H | H | H | O | $-CH_2-$ | 4-Phenyl | |
| 56 | $C_2H_5$ | H | H | H | O | $-CH_2-$ | H | |
| 57 | $C_2H_5$ | 3-Cl | H | H | O | $-CH_2-$ | H | |
| 58 | $SCH_3$ | 3-Cl | H | H | O | $-CH_2-$ | H | |
| 59 | $OCH_3$ | 3-Cl | H | H | O | $-CH_2-$ | H | |
| 60 | $OCH_3$ | 3-Cl | $CH_3$ | H | O | $-CH_2-$ | H | |
| 61 | $OCH_3$ | H | $CH_3$ | H | O | $-CH_2-$ | H | |
| 62 | $OCH_3$ | H | H | H | O | $-CH_2-$ | H | |
| 63 | $SCH_3$ | 3-Cl | $CH_3$ | H | O | $-CH_2-$ | H | |
| 64 | $SCH_3$ | H | $CH_3$ | H | O | $-CH_2-$ | H | |

| Nr. | $R^1$ | Y | $R^2$ | $R^3$ | X | A | Zn | Fp. [°C]/$n_D$ |
|---|---|---|---|---|---|---|---|---|
| 65 | $SCH_3$ | H | H | H | O | $-CH_2-$ | H | |
| 66 | $N(OCH_3)(CH_3)$ | H | H | H | O | $-CH_2-$ | 2,4-$Cl_2$ | |
| 68 | $N(OCH_3)(CH_3)$ | H | H | H | O | $-C(CH_3)_2-$ | H | |
| 69 | $N(OCH_3)(CH_3)$ | 3-Cl | H | H | O | $-CH_2-CH{=}CH-$ | H | |
| 70 | $N(OCH_3)(CH_3)$ | H | H | H | O | $-CH_2-C(CH_3){=}CH-$ | 4-$CH_3$ | |
| 71 | $N(OCH_3)(CH_3)$ | H | H | H | O | $-CH_2CH(CH_3)CH_2-$ | 4-$CH(CH_3)_2$ | |
| 72 | $N(OCH_3)(CH_3)$ | H | H | H | O | $-CH(C_2H_5)-$ | H | |
| 73 | $N(OCH_3)(CH_3)$ | H | H | H | O | $-CH(CH_3)CH_2-$ | H | |
| 74 | $N(OCH_3)(CH_3)$ | H | H | H | O | $-CH_2CH(CH_3)-$ | H | 60-61 |
| 75 | $N(OCH_3)(CH_3)$ | H | H | H | O | $-C(CH_3)_2CH_2-$ | H | |
| 76 | $N(OCH_3)(CH_3)$ | H | H | H | O | $-C(CH_3)_2CH_2-$ | 4-Cl | |
| 77 | $N(OCH_3)(CH_3)$ | H | H | H | O | $-CH_2CH(CH_3)CH_2-$ | 3,4-$Cl_2$ | |
| 79 | $N(OCH_3)(CH_3)$ | H | $CH_3$ | H | O | $-C(CH_3)_2-$ | H | |
| 80 | $N(OCH_3)(CH_3)$ | H | $CH_3$ | $C_2H_5$ | O | $-CH_2-$ | H | |
| 81 | $N(OCH_3)(CH_3)$ | H | H | H | $-N(CH_3)-$ | $-CH(CH_3)-$ | H | |
| 82 | $N(OC_2H_5)(CH_3)$ | 3-Cl | H | H | O | $-CH_2-$ | H | |
| 83 | $N(OCH_3((CH_3)$ | H | $CH_3$ | H | O | $-CH_2CH_2O-$ | H | |
| 84 | $N(OCH_3)(CH_3)$ | H | $CH_3$ | H | O | $-CH_2CH(CH_3)O-$ | H | |

| Nr. | $R^1$ | Y | $R^2$ | $R^3$ | X | A | Zn | Fp. [°C]/$n_D$ |
|---|---|---|---|---|---|---|---|---|
| 85 | $N(OCH_3)(CH_3)$ | H | $CH_3$ | H | O | $-CH_2CH_2O-$ | 4-Cl | |
| 86 | $N(OCH_3)(CH_3)$ | H | $CH_3$ | H | O | $-CH_2CH_2O-$ | 2,4-$Cl_2$ | 87-89 |
| 87 | $N(OCH_3)(CH_3)$ | H | $CH_3$ | H | O | $-CH_2CH(CH_3)O-$ | 2,4-$Cl_2$ | |
| 88 | $N(OCH_3)(CH_3)$ | H | H | H | O | $-CH_2CH_2O-$ | H | |
| 89 | $N(OCH_3)(CH_3)$ | H | H | H | O | $-CH_2CH_2O-$ | 4-Cl | |
| 90 | $N(OCH_3)(CH_3)$ | H | H | H | O | $-CH_2CH_2O-$ | 2,4-$Cl_2$ | |
| 91 | $N(OCH_3)(CH_3)$ | H | H | H | O | $-CH_2CH(CH_3)O-$ | H | $n_D^{20}$ = 1.5632 |
| 92 | $N(OCH_3)(CH_3)$ | H | H | H | O | $-CH_2CH(CH_3)O-$ | 4-Cl | |
| 93 | $N(OCH_3)(CH_3)$ | H | H | H | O | $-CH_2CH(CH_3)O-$ | 2,4-$Cl_2$ | |
| 94 | $N(OCH_3)(CH_3)$ | H | H | H | O | $-CH_2CH_2O-$ | 2,4,6-$Cl_3$ | |
| 95 | $N(OCH_3)(CH_3)$ | H | H | H | O | $-CH_2CH_2O-$ | 3-$CF_3$ | |
| 96 | $N(OCH_3)(CH_3)$ | H | H | $CH_3$ | O | $-CH_2CH_2O-$ | 3-$CF_3$ | |
| 97 | $N(OCH_3)(CH_3)$ | H | H | H | O | $-CH_2CH_2O-$ | 2-$CH_3$,4-Cl | |
| 98 | $N(OCH_3)(CH_3)$ | H | H | $CH_3$ | O | $-CH_2CH_2O-$ | 2-$CH_3$,4-Cl | |
| 99 | $N(OCH_3)(CH_3)$ | H | H | H | O | $-CH_2CH_2O-$ | 3-$OCH_3$ | |
| 100 | $N(OCH_3)(CH_3)$ | H | H | $CH_3$ | O | $-CH_2CH_2O-$ | 3-$OCH_3$ | |
| 101 | $N(OCH_3)(CH_3)$ | H | H | H | O | $-CH_2CH_2OCH_2CH_2-$ | H | |
| 102 | $N(OCH_3)(CH_3)$ | H | $CH_3$ | H | O | $-CH_2CH_2OCH_2CH_2-$ | H | |
| 103 | $N(OCH_3)(CH_3)$ | H | H | H | O | $-CH_2CH_2CH_2O-$ | H | |
| 104 | $N(OCH_3)(CH_3)$ | H | $CH_3$ | H | O | $-CH_2CH_2CH_2O-$ | H | |
| 105 | $N(OCH_3)(CH_3)$ | H | H | H | O | $-(CH_2)_4O-$ | H | |

| Nr. | $R^1$ | Y | $R^2$ | $R^3$ | X | A | Zn | Fp. [°C]/$n_D$ |
|---|---|---|---|---|---|---|---|---|
| 106 | $N(OCH_3)(CH_3)$ | H | $CH_3$ | H | O | $-(CH_2)_4O-$ | H | |
| 107 | $N(OCH_3)(CH_3)$ | H | H | H | O | $-CH_2CH_2OCH_2-$ | 2,4-$Cl_2$ | |
| 108 | $N(OCH_3)(CH_3)$ | H | $CH_3$ | H | O | $-CH_2CH_2OCH_2-$ | 2,4-$Cl_2$ | |
| 109 | $N(OCH_3)(CH_3)$ | H | H | H | O | $-CH_2CH_2OCH_2-$ | H | |
| 110 | $N(OCH_3)(CH_3)$ | H | $CH_3$ | H | O | $-CH_2CH_2OCH_2-$ | H | |
| 111 | $N(OCH_3)(CH_3)$ | H | H | H | O | $-(CH_2)_3OCH_2-$ | H | |
| 112 | $N(OCH_3)(CH_3)$ | H | $CH_3$ | H | O | $-(CH_2)_3OCH_2-$ | H | |
| 113 | $N(OCH_3)(CH_3)$ | 3-Cl | H | H | O | $-CH_2CH_2O-$ | H | |
| 114 | $N(OCH_3)(CH_3)$ | 3-Cl | $CH_3$ | H | O | $-CH_2CH_2O-$ | H | |
| 115 | $N(OCH_3)(CH_3)$ | 3-Cl | H | H | O | $-CH_2CH(CH_3)O-$ | H | |
| 116 | $N(OCH_3)(CH_3)$ | 3-Cl | $CH_3$ | H | O | $-CH_2CH(CH_3)O-$ | H | |
| 117 | $N(OCH_3)(CH_3)$ | 3-Cl | H | H | O | $-CH_2CH_2O-$ | 2,4-$Cl_2$ | |
| 118 | $N(OCH_3)(CH_3)$ | 3-Cl | $CH_3$ | H | O | $-CH_2CH_2O-$ | 2,4-$Cl_2$ | |
| 119 | $N(OCH_3)(CH_3)$ | 3-Cl | $CH_3$ | $CH_3$ | O | $-CH_2CH_2O-$ | 2,4-$Cl_2$ | |
| 120 | $N(OCH_3)(CH_3)$ | 3-Cl | H | H | O | $-CH_2CH(CH_3)O-$ | 2,4-$Cl_2$ | |
| 121 | $N(OCH_3)(CH_3)$ | 3-Cl | $CH_3$ | H | O | $-CH_2CH(CH_3)O-$ | 2,4-$Cl_2$ | |
| 122 | $N(OCH_3)(CH_3)$ | 3-Cl | $CH_3$ | $CH_3$ | O | $-CH_2CH(CH_3)O-$ | 2,4-$Cl_2$ | |
| 123 | $N(CH_3)_2$ | H | H | H | O | $-CH_2-CH_2-$ | H | 96-98 |
| 124 | $N(CH_3)_2$ | H | H | H | O | $-CH_2CH_2O-$ | H | |
| 125 | $N(CH_3)_2$ | H | $CH_3$ | H | O | $-CH_2CH_2O-$ | H | |
| 126 | $N(CH_3)_2$ | H | H | H | O | $-CH_2CH_2O-$ | 4-Cl | |

| Nr. | $R^1$ | Y | $R^2$ | $R^3$ | X | A | Zn | Fp. [°C]/$n_D$ |
|---|---|---|---|---|---|---|---|---|
| 127 | $N(CH_3)_2$ | H | $CH_3$ | H | O | $-CH_2CH_2O-$ | 4-Cl | |
| 128 | $N(CH_3)_2$ | H | H | H | O | $-CH_2CH_2O-$ | 2,4-$Cl_2$ | |
| 129 | $N(CH_3)_2$ | H | $CH_3$ | H | O | $-CH_2CH_2O-$ | 2,4-$Cl_2$ | |
| 130 | $N(CH_3)_2$ | H | H | H | O | $-CH_2CH(CH_3)O-$ | H | |
| 131 | $N(CH_3)_2$ | H | $CH_3$ | H | O | $-CH_2CH(CH_3)O-$ | H | |
| 132 | $N(CH_3)_2$ | 3-Cl | H | H | O | $-CH_2CH(CH_3)O-$ | H | |
| 133 | $N(CH_3)_2$ | 3-Cl | $CH_3$ | H | O | $-CH_2CH(CH_3)O-$ | H | |
| 134 | $N(CH_3)_2$ | H | H | H | O | $-CH(CH_3)CH_2O-$ | H | |
| 135 | $N(CH_3)_2$ | 3-Cl | H | H | O | $-CH(CH_3)CH_2O-$ | H | |
| 136 | $N(OCH_3)(CH_3)$ | H | H | H | O | $-CH_2-$ | 3,4-$Cl_2$ | 47-49 |
| 137 | $N(CH_3)_2$ | H | H | H | O | $-CH_2-$ | 3,4-$Cl_2$ | |
| 138 | $N(CH_3)_2$ | H | $CH_3$ | H | O | $-CH_2-$ | 3,4-$Cl_2$ | |
| 139 | $N(OCH_3)(CH_3)$ | H | $CH_3$ | H | O | $-CH_2-$ | 3,4-$Cl_2$ | |
| 140 | $N(OCH_3)(CH_3)$ | 3-Cl | H | H | O | $-CH_2-$ | 3,4-$Cl_2$ | |
| 141 | $N(OCH_3)(CH_3)$ | 3-Cl | $CH_3$ | H | O | $-CH_2-$ | 3,4-$Cl_2$ | |
| 142 | $N(CH_3)_2$ | 3-Cl | H | H | O | $-CH_2-$ | H | 88-95 |
| 143 | $N(CH_3)_2$ | H | $CH_3$ | H | O | $-CH_2-$ | 4-$CH_3$ | |
| 144 | $N(CH_3)_2$ | 3-Cl | $CH_3$ | H | O | $-CH_2-$ | H | 107-109 |
| 145 | Ethyl | H | H | H | O | $-CH_2CH_2O-$ | H | |
| 146 | Ethyl | H | $CH_3$ | H | O | $-CH_2CH_2O-$ | H | |
| 147 | Ethyl | H | H | H | O | $-CH_2CH_2O-$ | 2,4-$Cl_2$ | |

| Nr. | $R^1$ | Y | $R^2$ | $R^3$ | X | A | Zn | Fp. [°C]/$n_D$ |
|---|---|---|---|---|---|---|---|---|
| 148 | Ethyl | H | $CH_3$ | H | O | $-CH_2CH_2O-$ | 2,4-$Cl_2$ | |
| 149 | Ethyl | 3-Cl | H | H | O | $-CH_2CH_2O-$ | H | |
| 150 | Ethyl | 3-Cl | $CH_3$ | H | O | $-CH_2CH_2O-$ | H | |
| 151 | Ethyl | 3-Cl | H | H | O | $-CH_2CH_2O-$ | 2,4-$Cl_2$ | |
| 152 | Ethyl | 3-Cl | $CH_3$ | H | O | $-CH_2CH_2O-$ | 2,4-$Cl_2$ | |
| 153 | $N(OCH_3)(CH_3)$ | 3-Cl | H | H | O | $-CH_2CH_2OCH_2-$ | H | |
| 154 | $N(OCH_3)(CH_3)$ | 3-Cl | $CH_3$ | H | O | $-CH_2CH_2OCH_2-$ | H | |
| 155 | $N(OCH_3)(CH_3)$ | 3-Cl | H | H | O | $-(CH_2)_3OCH_2-$ | H | |
| 156 | $N(OCH_3)(CH_3)$ | 3-Cl | $CH_3$ | H | O | $-(CH_2)_3OCH_2-$ | H | |
| 157 | $N(CH_3)_2$ | H | H | H | O | $-(CH_2)_3OCH_2-$ | H | |
| 158 | $N(CH_3)_2$ | H | H | H | O | $-CH_2CH_2OCH_2-$ | H | |
| 159 | $N(OCH_3)(CH_3)$ | H | $CH_3$ | H | O | $-CH_2-$ | 4-(2-Cl-4-$CF_3$-phenoxy) | |
| 160 | $N(OCH_3)(CH_3)$ | H | H | H | O | $-CH_2-$ | 4-(2-Cl-4-$CF_3$-phenoxy) | |
| 161 | $N(OCH_3)(CH_3)$ | 3-Cl | H | H | O | $-CH_2-$ | 4-(2-Cl-4-$CF_3$-phenoxy) | |
| 162 | $N(OCH_3)(CH_3)$ | H | $CH_3$ | H | O | $-CH_2-$ | 3-(2-Cl-4-$CF_3$-phenoxy) | |
| 163 | $N(OCH_3)(CH_3)$ | H | H | H | O | $-CH_2-$ | 3-(2-Cl-4-$CF_3$-phenoxy) | |
| 164 | $N(OCH_3)(CH_3)$ | 3-Cl | H | H | O | $-CH_2-$ | 3-(2-Cl-4-$CF_3$-phenoxy) | |
| 165 | $N(OCH_3)(CH_3)$ | H | H | H | O | $-CH_2-$ | 4-$CF_3$ | 61-62 |
| 166 | $N(OCH_3)(CH_3)$ | H | H | H | O | $-CH_2-$ | 3-(4-$CF_3$-phenoxy) | |
| 167 | $N(CH_3)_2$ | H | H | H | O | $-CH_2-$ | 4-(2,4-$Cl_2$-phenoxy) | |
| 168 | $N(CH_3)_2$ | H | H | H | O | $-CH_2-$ | 3-(2,4-$Cl_2$-phenoxy) | |

| Nr. | $R^1$ | Y | $R^2$ | $R^3$ | X | A | Zn | Fp. [°C]/$n_D$ |
|---|---|---|---|---|---|---|---|---|
| 169 | $N(OCH_3)(CH_3)$ | H | H | H | O | $-CH_2-$ | 4-(2,4-$Cl_2$-phenoxy) | |
| 170 | $N(OCH_3)(CH_3)$ | H | H | H | O | $-CH_2-$ | 3-(2,4-$Cl_2$-phenoxy) | |
| 171 | Ethyl | H | $CH_3$ | H | O | $-CH_2CH_2OCH_2-$ | H | |
| 172 | Ethyl | H | H | H | O | $-CH_2CH_2OCH_2-$ | H | |
| 173 | Ethyl | H | H | H | O | $-(CH_2)_3OCH_2$ | H | |
| 174 | Ethyl | H | $CH_3$ | H | O | $-(CH_2)_3OCH_2$ | H | |
| 175 | $N(CH_3)_2$ | H | H | H | O | $-CH_2-$ | 4-i-$C_3H_7$ | 87-89 |
| 176 | $N(OCH_3)(CH_3)$ | H | H | H | O | $-CH_2-$ | 4-i-$C_3H_7$ | 62-63 |
| 177 | $N(OCH_2)(CH_3)$ | H | H | H | O | $-CH(CH_3)-$ | 4-Phenyl | |
| 178 | $N(CH_3)_2$ | H | H | H | O | $-CH(CH_3)-$ | 4-Phenyl | |

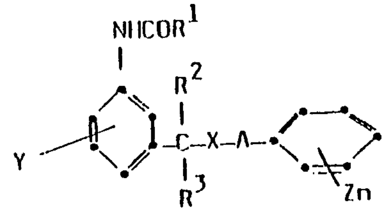

| Nr. | $R^1$ | Y | $R^2$ | $R^3$ | X | A | Zn | Fp. [°C]/$n_D$ |
|---|---|---|---|---|---|---|---|---|
| 179 | $NHCH_3$ | H | H | H | O | $-CH_2-$ | H | 106-107 |
| 180 | $NHC_2H_5$ | H | H | H | O | $-CH_2-$ | H | 65- 68 |
| 181 | 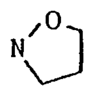 | H | H | H | O | $-CH_2-$ | H | |
| 182 | 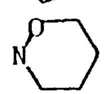 | H | H | H | O | $-CH_2-$ | H | |
| 183 | $C_2H_5$ | H | H | H | O | $-CH_2-$ | H | $n_D^{20}$ = 1.5769 |
| 184 | $SCH_3$ | H | H | H | O | $-CH_2-$ | H | 82- 83 |
| 185 | $OCH_3$ | H | H | H | O | $-CH_2-$ | H | $n_D^{25}$ = 1.5738 |
| 186 | $OC_2H_5$ | H | H | H | O | $-CH_2-$ | H | $n_D^{20}$ = 1.5665 |
| 187 | Cyclopropyl | H | H | H | O | $-CH_2-$ | H | 87- 88 |
| 188 | $t-C_4H_9$ | H | H | H | O | $-CH_2-$ | H | 102-103 |

| Nr. | $R^1$ | Y | $R^2$ | $R^3$ | X | A | Zn | Fp. [°C]/$n_D$ |
|---|---|---|---|---|---|---|---|---|
| 189 | $N(C_2H_5)_2$ | H | H | H | O | $-CH_2-$ | H | 99-100 |
| 190 | $NHC_3H_7$ | H | H | H | O | $-CH_2-$ | H | 74- 75 |
| 191 | N-Methyl-N--cyclohexyl-amino | H | H | H | O | $-CH_2-$ | H | |
| 192 | 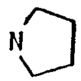 | H | H | H | O | $-CH_2-$ | H | 103-104 |
| 193 | 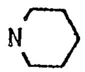 | H | H | H | O | $-CH_2-$ | H | 119-120 |
| 194 | 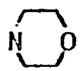 | H | $CH_3$ | H | O | $-CH_2-$ | 4-Cl | 116-117 |
| 195 | N-Cyclopropylamino | H | $CH_3$ | H | O | $-CH_2CH_2-$ | H | |
| 196 | $N(nC_4H_9)C_2H_5$ | H | $CH_3$ | H | O | $-CH(CH_3)-$ | H | |
| 197 | $NH(OC_2H_5)$ | H | H | H | O | $-CH_2-$ | 4-Cl | |
| 198 | $NH(CH_2CH=CH_2)$ | H | H | H | O | $-CH_2-CH_2-$ | H | |
| 199 | $NHC(CH_3)_2C{\equiv}CH$ | H | H | H | O | $-CH_2-$ | 4-F | |
| 200 | $O-i-C_3H_7$ | H | $CH_3$ | H | O | $-C(CH_3)_2-$ | H | |
| 201 | $O-sec-C_4H_9$ | H | H | H | O | $-CH_2-$ | H | 38- 39 |
| 202 | $O-CH(CH_3)C{\equiv}CH$ | H | H | H | O | $-CH_2-$ | $3-CH_3$ | |

| Nr. | $R^1$ | Y | $R^2$ | $R^3$ | X | A | Zn | Fp. [°C]/$n_D$ |
|---|---|---|---|---|---|---|---|---|
| 203 | $O-CH_2CH=CH_2$ | H | H | H | O | $-CH_2-$ | $2-CH_3$ | |
| 204 | Cyclohexoxy | H | H | H | O | $-CH_2-$ | H | 53-56 |
| 205 | $SC_2H_5$ | H | H | H | O | $-CH_2-$ | H | 51-52 |
| 206 | $CCl_3$ | H | H | H | O | $-CH_2-$ | H | 73-74 |
| 207 | $CHCl_2$ | H | H | H | O | $-CH_2-$ | H | 92-93 |
| 208 | $CH_2Cl$ | H | H | H | O | $-CH_2-$ | H | 54-55 |
| 209 | $CCl_2CH_3$ | H | H | H | O | $-CH_2-$ | H | 84-86 |
| 210 | $C(CH_3)=CH_2$ | H | $CH_3$ | H | O | $-CH_2-$ | H | |
| 211 | $CH_2CH(CH_3)CH_3$ | H | H | H | O | $-CH_2-$ | H | 87-88 |
| 212 | sec.-$C_5H_{11}$ | H | H | H | O | $-CH_2-$ | H | 58-59 |
| 213 | Cyclohexyl | H | H | H | O | $-CH_2-$ | H | 89-90 |
| 214 | $N(CH_3)_2$ | H | H | H | O | $-CH_2-$ | $2,4-Cl_2$ | |
| 215 | $N(CH_3)_2$ | H | $C_2H_5$ | H | O | $-CH_2-$ | H | |
| 216 | $N(CH_3)_2$ | H | H | H | O | $-CH_2-$ | $2,3,6-Cl_3$ | |
| 217 | $N(CH_3)_2$ | H | H | H | O | $-CH_2-$ | $2,6-Cl_2$ | |
| 218 | $N(CH_3)_2$ | H | $CH_3$ | H | $-N(CH_3)-$ | $-CH_2-$ | H | |
| 219 | $N(CH_3)_2$ | H | H | H | O | $-CH_2-$ | $4-i-C_3H_7$ | 121-123 |
| 220 | $N(CH_3)_2$ | H | H | H | O | $-CH_2CH(CH_3)-CH_2-CH(CH_3)-CH_2-$ | H | |
| 221 | $N(CH_3)_2$ | H | H | H | O | $-CH_2-$ | $4-CF_3$ | |
| 222 | $N(CH_3)_2$ | H | H | H | O | $-CH_2-$ | $4-Cl$ | |
| 223 | $N(CH_3)_2$ | H | H | H | O | $-CH_2-$ | $4-O-t-C_4H_9$ | |

| Nr. | $R^1$ | Y | $R^2$ | $R^3$ | X | A | Zn | Fp. [°C]/$n_D$ |
|---|---|---|---|---|---|---|---|---|
| 224 | $OCH_3$ | H | H | H | O | $-CH_2-$ | $3-CH_3$ | |
| 225 | $OCH_3$ | H | H | H | O | $-CH_2-$ | 4-Cl | |
| 226 | $OCH_3$ | H | $CH_3$ | H | O | $-CH_2-$ | 4-Cl | 65- 66 |
| 227 | $OCH_3$ | H | $CH_3$ | H | O | $-CH_2-$ | H | 77- 78 |
| 229 | $SCH_3$ | H | H | H | O | $-CH_2-$ | $3-CH_3$ | |
| 230 | $SCH_3$ | H | H | H | O | $-CH_2-$ | $4-CH_3$ | |
| 231 | $SCH_3$ | H | H | H | O | $-CH_2-$ | 4-Cl | |
| 232 | $SCH_3$ | H | H | H | O | $-CH_2-$ | 3-Cl | |
| 233 | $SCH_3$ | H | H | H | O | $-CH_2CH_2-$ | H | |
| 234 | $C_2H_5$ | H | H | H | O | $-CH_2-$ | $3-CH_3$ | |
| 235 | $C_2H_5$ | H | H | H | O | $-CH_2-$ | $4-CH_3$ | 68 |
| 236 | $C_2H_5$ | H | H | H | O | $-CH_2-$ | 4-Cl | 82- 84 |
| 237 | $C_2H_5$ | H | H | H | O | $-CH_2-$ | $2,4-Cl_2$ | |
| 238 | $C_2H_5$ | H | H | H | O | $-CH_2-$ | 3-Cl | |
| 239 | $C_2H_5$ | H | H | H | O | $-CH_2CH_2-$ | H | |

| Nr. | $R^1$ | Y | $R^2$ | $R^3$ | X | A | Zn | Fp. [°C]/$n_D$ |
|---|---|---|---|---|---|---|---|---|
| 244 | $C_2H_5$ | H | H | H | $-N(CH_3)-$ | $-CH_2-$ | H | |
| 245 | $C_2H_5$ | H | $CH_3$ | H | $-N(CH_3)-$ | $-CH_2-$ | H | |
| 246 | $C_2H_5$ | H | H | $CH_3$ | O | $-CH_2-$ | H | 55- 57 |
| 247 | $C_2H_5$ | H | $CH_3$ | H | O | $-CH_2-$ | 3-$CH_3$ | |
| 248 | $C_2H_5$ | H | H | H | O | $-CH(CH_3)-$ | H | |
| 249 | $C_2H_5$ | H | H | H | O | $-CH(CH_3)-$ | 4-$CH_3$ | |
| 250 | $C_2H_5$ | H | H | $CH_3$ | O | $-CH_2-$ | 4-$CH_3$ | $n_D^{20}$ = 1.5627 |
| 251 | $C_2H_5$ | H | $CH_3$ | H | O | $-CH_2-$ | 4-Cl | $n_D^{20}$ = 1.5706 |
| 252 | $C_2H_5$ | H | $C_2H_5$ | H | O | $-CH_2-$ | H | |
| 253 | $C_2H_5$ | H | H | $C_2H_5$ | O | $-CH_2-$ | 4-Cl | |
| 254 | $C_2H_5$ | H | H | H | O | $-CH_2CH(CH_3)CH_2-$ | 4-Cl | |
| 255 | $C_2H_5$ | H | H | H | O | $-CH(C_2H_5)-$ | H | |
| 256 | $C_2H_5$ | H | H | H | $-N(CH_3)-$ | $-CH(CH_3)-$ | H | |
| 257 | $C_2H_5$ | H | H | H | $-N(C_3H_7)-$ | $-CH_2-$ | H | |
| 258 | $SCH_3$ | H | H | H | O | $-CH_2CH(CH_3)CH_2-$ | 4-Cl | |
| 259 | $OCH_3$ | H | H | H | O | $-CH_2CH(CH_3)CH_2-$ | 4-Cl | |
| 260 | $N(OCH_3)(CH_3)$ | H | H | H | O | $-CH_2CH(CH_3)CH_2-$ | 4-Cl | |
| 261 | $N(CH_3)_2$ | H | H | H | O | $-CH_2CH(CH_3)CH_2-$ | 4-Cl | |
| 262 | $N(OCH_3)(CH_3)$ | H | H | H | O | $-CH_2-$ | H | 50- 51 |
| 263 | $N(OCH_3)(CH_3)$ | H | H | H | O | $-CH_2-$ | 4-$CH_3$ | 55-57 |

| Nr. | $R^1$ | Y | $R^2$ | $R^3$ | X | A | Zn | Fp. [°C]/$n_D$ |
|---|---|---|---|---|---|---|---|---|
| 264 | $N(OCH_3)(CH_3)$ | H | H | H | O | $-CH_2-$ | 4-Cl | |
| 265 | $N(OCH_3)(CH_3)$ | H | H | H | O | $-CH_2-$ | 2,4-$Cl_2$ | |
| 266 | $N(OCH_3)(CH_3)$ | H | $CH_3$ | H | O | $-CH_2-$ | H | $n_D^{20}$ = 1.5671 |
| 267 | $N(OCH_3)(CH_3)$ | H | H | $CH_3$ | O | $-CH_2-$ | 4-Cl | $n_D^{20}$ = 1.5698 |
| 268 | $N(OCH_3)(CH_3)$ | H | $CH_3$ | H | O | $-CH_2-$ | 4-$CH_3$ | $n_D^{20}$ = 1.5605 |
| 269 | $N(OCH_3)(CH_3)$ | H | $C_2H_5$ | H | O | $-CH_2-$ | H | |
| 270 | $N(OCH_3)(CH_3)$ | H | H | $C_2H_5$ | O | $-CH_2-$ | 4-Cl | |
| 271 | $N(OCH_3)(CH_3)$ | H | H | H | O | $-CH(CH_3)-$ | H | |
| 272 | $N(OCH_3)(CH_3)$ | H | H | H | O | $-CH_2CH_2-$ | H | |
| 273 | $N(OCH_3)(CH_3)$ | H | H | H | O | $-CH_2CH_2-$ | 4-Cl | |
| 274 | $N(OCH_3)(CH_3)$ | H | H | H | O | $-CH_2CH_2-$ | 4-$CH_3$ | |
| 276 | $N(OCH_3)(CH_3)$ | H | H | H | O | $-CH_2-$ | 4-i-$C_3H_7$ | 73-74 |
| 279 | $N(OCH_3)(CH_3)$ | H | H | H | $-N(CH_3)-$ | $-CH_2-$ | H | |
| 280 | $N(OCH_3)(CH_3)$ | H | H | H | O | $-C(CH_3)_2-$ | 4-Cl | |
| 281 | $N(OCH_3)(CH_3)$ | H | H | H | O | $-CH_2CH(CH_3)-$ | H | $n_D^{20}$ = 1.5641 |
| 282 | $N(OCH_3)(CH_3)$ | H | H | H | O | $-CH_2CH{=}CH-$ | H | |
| 283 | $N(OCH_3)(CH_3)$ | H | $CH_3$ | H | $-N(C_5H_{11})$ | $-CH_2-$ | H | |

| Nr. | $R^1$ | Y | $R^2$ | $R^3$ | X | A | Zn | Fp. [°C]/$n_D$ |
|---|---|---|---|---|---|---|---|---|
| 284 | $N(OCH_3)(CH_3)$ | H | $CH_3$ | H | O | $-CH_2-CH_2-O-$ | H | |
| 285 | $N(OCH_3)(CH_3)$ | H | $CH_3$ | H | O | $-CH_2-CH(CH_3)-O-$ | H | |
| 286 | $N(OCH_3)(CH_3)$ | H | $CH_3$ | H | O | $-CH_2-CH_2-O-$ | 4-Cl | |
| 287 | $N(OCH_3)(CH_3)$ | H | $CH_3$ | H | O | $-CH_2-CH_2-O-$ | 2,4-$Cl_2$ | |
| 288 | $N(OCH_3)(CH_3)$ | H | $CH_3$ | H | O | $-CH_2-CH(CH_3)-O-$ | 2,4-$Cl_2$ | |
| 289 | $N(OCH_3)(CH_3)$ | H | H | H | O | $-CH_2-CH_2-O-$ | H | |
| 290 | $N(OCH_3)(CH_3)$ | H | H | H | O | $-CH_2-CH_2-O-$ | 4-Cl | |
| 291 | $N(OCH_3)(CH_3)$ | H | H | H | O | $-CH_2-CH_2-O-$ | 2,4-$Cl_2$ | |
| 292 | $N(OCH_3)(CH_3)$ | H | H | H | O | $-CH_2-CH(CH_3)-O-$ | H | 53- 56 |
| 293 | $N(OCH_3)(CH_3)$ | H | H | H | O | $-CH_2-CH(CH_3)-O-$ | 4-Cl | |
| 294 | $N(OCH_3)(CH_3)$ | H | H | H | O | $-CH_2-CH(CH_3)-O-$ | 2,4-$Cl_2$ | |
| 295 | $N(OCH_3)(CH_3)$ | H | H | H | O | $-CH_2-CH_2-O-$ | 2,4,6-$Cl_3$ | 44- 46 |
| 296 | $N(OCH_3)(CH_3)$ | H | H | H | O | $-CH_2-CH_2-O-$ | 3-$CF_3$ | |
| 297 | $N(OCH_3)(CH_3)$ | H | H | $CH_3$ | O | $-CH_2-CH_2-O-$ | 3-$CF_3$ | |
| 298 | $N(OCH_3)(CH_3)$ | H | H | H | O | $-CH_2-CH_2-O-$ | 2-$CH_3$, 4-Cl | |
| 299 | $N(OCH_3)(CH_3)$ | H | H | $CH_3$ | O | $-CH_2-CH_2-O-$ | 2-$CH_3$, 4-Cl | |
| 300 | $N(OCH_3)(CH_3)$ | H | H | H | O | $-CH_2-CH_2-O-$ | 3-$OCH_3$ | |
| 301 | $N(OCH_3)(CH_3)$ | H | H | $CH_3$ | O | $-CH_2-CH_2-O-$ | 3-$OCH_3$ | |

| Nr. | $R^1$ | Y | $R^2$ | $R^3$ | X | A | Zn | Fp. [°C]/$n_D$ |
|---|---|---|---|---|---|---|---|---|
| 302 | $N(OCH_3)(CH_3)$ | H | H | H | O | $-CH_2-CH_2-O-CH_2-CH_2$ | H | |
| 303 | $N(OCH_3)(CH_3)$ | H | $CH_3$ | H | O | $-CH_2-CH_2-O-CH_2-CH_2$ | H | |
| 304 | $N(OCH_3)(CH_3)$ | H | H | H | O | $-CH_2-CH_2-CH_2-O-$ | H | |
| 305 | $N(OCH_3)(CH_3)$ | H | $CH_3$ | H | O | $-CH_2-CH_2-CH_2-O-$ | H | |
| 306 | $N(OCH_3)(CH_3)$ | H | H | H | O | $-(CH_2)_4-O-$ | H | |
| 307 | $N(OCH_3)(CH_3)$ | H | $CH_3$ | H | O | $-(CH_2)_4-O-$ | H | |
| 308 | $N(OCH_3)(CH_3)$ | H | H | H | O | $-CH_2-CH_2-O-CH_2-$ | 2,4-$Cl_2$ | |
| 309 | $N(OCH_3)(CH_3)$ | H | $CH_3$ | H | O | $-CH_2-CH_2-O-CH_2-$ | 2,4-$Cl_2$ | |
| 310 | $N(OCH_3)(CH_3)$ | H | H | H | O | $-CH_2-CH_2-O-CH_2-$ | H | |
| 311 | $N(OCH_3)(CH_3)$ | H | $CH_3$ | H | O | $-CH_2-CH_2-O-CH_2-$ | H | |
| 312 | $N(OCH_3)(CH_3)$ | H | H | H | O | $-(CH_2)_3-O-CH_2-$ | H | |
| 313 | $N(OCH_3)(CH_3)$ | H | $CH_3$ | H | O | $-(CH_2)_3-O-CH_2-$ | H | |
| 314 | $N(OCH_3)(CH_3)$ | 4-Cl | H | H | O | $-CH_2-CH_2-O-$ | H | |
| 315 | $N(OCH_3)(CH_3)$ | 4-Cl | $CH_3$ | H | O | $-CH_2-CH_2-O-$ | H | |
| 316 | $N(OCH_3)(CH_3)$ | 4-Cl | H | H | O | $-CH_2-CH(CH_3)-O-$ | H | |
| 317 | $N(OCH_3)(CH_3)$ | 4-Cl | $CH_3$ | H | O | $-CH_2-CH(CH_3)-O-$ | H | |
| 318 | $N(OCH_3)(CH_3)$ | 4-Cl | H | H | O | $-CH_2-CH_2-O-$ | 2,4-$Cl_2$ | |
| 319 | $N(OCH_3)(CH_3)$ | 4-Cl | $CH_3$ | H | O | $-CH_2-CH_2-O-$ | 2,4-$Cl_2$ | |
| 320 | $N(OCH_3)(CH_3)$ | 4-Cl | $CH_3$ | $CH_3$ | O | $-CH_2-CH_2-O-$ | 2,4-$Cl_2$ | |

| Nr. | $R^1$ | Y | $R^2$ | $R^3$ | X | A | Zn | Fp. [°C]/$n_D$ |
|---|---|---|---|---|---|---|---|---|
| 321 | $N(OCH_3)(CH_3)$ | 4-Cl | H | H | O | $-CH_2-CH(CH_3)-O-$ | 2,4-$Cl_2$ | |
| 322 | $N(OCH_3)(CH_3)$ | 4-Cl | $CH_3$ | H | O | $-CH_2-CH(CH_3)-O-$ | 2,4-$Cl_2$ | |
| 323 | $N(OCH_3)(CH_3)$ | 4-Cl | $CH_3$ | $CH_3$ | O | $-CH_2-CH(CH_3)-O-$ | 2,4-$Cl_2$ | |
| 324 | $N(CH_3)_2$ | H | H | H | O | $-CH_2-CH_2-$ | H | |
| 325 | $N(CH_3)_2$ | H | H | H | O | $-CH_2-CH_2-O-$ | H | |
| 326 | $N(CH_3)_2$ | H | $CH_3$ | H | O | $-CH_2-CH_2-O-$ | H | |
| 327 | $N(CH_3)_2$ | H | H | H | O | $-CH_2-CH_2-O-$ | 4-Cl | |
| 328 | $N(CH_3)_2$ | H | $CH_3$ | H | O | $-CH_2-CH_2-O-$ | 4-Cl | |
| 329 | $N(CH_3)_2$ | H | H | H | O | $-CH_2-CH_2-O-$ | 2,4-$Cl_2$ | |
| 330 | $N(CH_3)_2$ | H | $CH_3$ | H | O | $-CH_2-CH_2-O-$ | 2,4-$Cl_2$ | |
| 331 | $N(CH_3)_2$ | H | H | H | O | $-CH_2-CH(CH_3)-O-$ | H | |
| 332 | $N(CH_3)_2$ | H | $CH_3$ | H | O | $-CH_2-CH(CH_3)-O-$ | H | |
| 333 | $N(CH_3)_2$ | 4-Cl | H | H | O | $-CH_2-CH(CH_3)-O-$ | H | |
| 334 | $N(CH_3)_2$ | 4-Cl | $CH_3$ | H | O | $-CH_2-CH(CH_3)-O-$ | H | |
| 335 | $N(CH_3)_2$ | H | H | H | O | $-CH(CH_3)-CH_2-O-$ | H | |
| 336 | $N(CH_3)_2$ | 4-Cl | H | H | O | $-CH(CH_3)-CH_2-O-$ | H | |
| 337 | $N(OCH_3)(CH_3)$ | H | H | H | O | $-CH_2-$ | 3,4-$Cl_2$ | |
| 338 | $N(CH_3)_2$ | H | H | H | O | $-CH_2-$ | 3,4-$Cl_2$ | |
| 339 | $N(CH_3)_2$ | H | $CH_3$ | H | O | $-CH_2-$ | 3,4-$Cl_2$ | |
| 340 | $N(OCH_3)(CH_3)$ | H | $CH_3$ | H | O | $-CH_2-$ | 3,4-$Cl_2$ | |

| Nr. | $R^1$ | Y | $R^2$ | $R^3$ | X | A | Zn | Fp. [°C]/$n_D$ |
|---|---|---|---|---|---|---|---|---|
| 341 | $N(OCH_3)(CH_3)$ | 4-Cl | H | H | O | $-CH_2-$ | 3,4-$Cl_2$ | |
| 342 | $N(OCH_3)(CH_3)$ | 4-Cl | $CH_3$ | H | O | $-CH_2-$ | 3,4-$Cl_2$ | |
| 343 | $N(CH_3)_2$ | 4-Cl | H | H | O | $-CH_2-$ | 3,4-$Cl_2$ | |
| 344 | $N(CH_3)_2$ | H | H | H | O | $-CH_2-$ | 4-$CH_3$ | 92-94 |
| 345 | $N(CH_3)_2$ | 4-Cl | $CH_3$ | H | O | $-CH_2-$ | H | |
| 346 | Ethyl | H | H | H | O | $-CH_2-CH_2-O-$ | H | |
| 347 | Ethyl | H | $CH_3$ | H | O | $-CH_2-CH_2-O-$ | H | |
| 348 | Ethyl | H | H | H | O | $-CH_2-CH_2-O-$ | 2,4-$Cl_2$ | |
| 349 | Ethyl | H | $CH_3$ | H | O | $-CH_2-CH_2-O-$ | 2,4-$Cl_2$ | |
| 350 | Ethyl | 4-Cl | H | H | O | $-CH_2-CH_2-O-$ | H | |
| 351 | Ethyl | 4-Cl | $CH_3$ | H | O | $-CH_2-CH_2-O-$ | H | |
| 352 | Ethyl | 4-Cl | H | H | O | $-CH_2-CH_2-O-$ | 2,4-$Cl_2$ | |
| 353 | Ethyl | 4-Cl | $CH_3$ | H | O | $-CH_2-CH_2-O-$ | 2,4-$Cl_2$ | |
| 354 | $N(OCH_3)(CH_3)$ | 4-Cl | H | H | O | $-CH_2-CH_2-O-CH_2$ | H | |
| 355 | $N(OCH_3)(CH_3)$ | 4-Cl | $CH_3$ | H | O | $-CH_2-CH_2-O-CH_2$ | H | |
| 356 | $N(OCH_3)(CH_3)$ | 4-Cl | H | H | O | $-(CH_2)_3-O-CH_2$ | H | |
| 357 | $N(OCH_3)(CH_3)$ | H | H | H | O | $-CH_2-$ | 4-$CF_3$ | |
| 358 | $N(CH_3)_2$ | H | H | H | O | $-(CH_2)_3-O-CH_2$ | H | |
| 359 | $N(CH_3)_2$ | H | H | H | O | $-CH_2-CH_2-O-CH_2$ | H | |
| 360 | $N(CH_3)_2$ | H | H | H | O | $-CH_2-$ | H | 113-116 |

| Nr. | $R^1$ | Y | $R^2$ | $R^3$ | X | A | Zn | Fp. [°C]/$n_D$ |
|---|---|---|---|---|---|---|---|---|
| 361 | $N(CH_3)_2$ | H | $CH_3$ | H | O | $-CH_2-$ | H | 73- 75 |
| 362 | $N(CH_3)_2$ | H | $CH_3$ | H | O | $-CH_2-$ | 4-$CH_3$ | 73- 74 |
| 363 | $N(CH_3)_2$ | H | $CH_3$ | H | O | $-CH_2-$ | 4-Cl | Öl |
| 364 | $N(CH_3)_2$ | H | H | H | O | $-CH_2-$ | 4-(2-Cl-4-$CF_3$-phenoxy) | |
| 365 | $N(CH_3)_2$ | H | H | H | O | $-CH_2-$ | 3-(2-Cl-4-$CF_3$-phenoxy) | |
| 366 | $N(CH_3)_2$ | H | H | H | O | $-CH_2-$ | 4-(2,4-$Cl_2$-phenoxy) | |
| 367 | $N(CH_3)_2$ | H | H | H | O | $-CH_2-$ | 3-(2,4-$Cl_2$-phenoxy) | |
| 368 | $-C(CH_3)=CH_2$ | H | $CH_3$ | H | O | $-CH_2-$ | H | 47- 48 |
| 369 | $N(OCH_3)(CH_3)$ | H | H | H | O | $-CH_2-$ | 4-(2-Cl-4-$CF_3$-phenoxy) | 58- 62 |
| 370 | $N(OCH_3)(CH_3)$ | H | $CH_3$ | H | O | $-CH_2-$ | 4-(2-Cl-4-$CF_3$-phenoxy) | |
| 371 | $N(OCH_3)(CH_3)$ | 4-Cl | H | H | O | $-CH_2-$ | 4-(2-Cl-4-$CF_3$-phenoxy) | |
| 372 | $N(OCH_3)(CH_3)$ | H | H | H | O | $-CH_2-$ | 3-(2-Cl-4-$CF_3$-phenoxy) | $n_D^{20}$ = 1.5616 |
| 373 | $N(OCH_3)(CH_3)$ | H | $CH_3$ | H | O | $-CH_2-$ | 3-(2-Cl-4-$CF_3$-phenoxy) | |
| 374 | $N(OCH_3)(CH_3)$ | 4-Cl | H | H | O | $-CH_2-$ | 3-(2-Cl-4-$CF_3$-phenoxy) | |
| 375 | $N(OC_2H_5)(C_2H_5)$ | H | H | H | O | $-CH_2-$ | H | $n_D^{20}$ = 1.5620 |
| 376 | NH-Cyclopropyl | H | $CH_3$ | H | O | $-CH_2-$ | 4-Cl | zähes Öl |
| 377 | Ethyl | H | H | H | O | $-CH_2-CH_2-O-CH_2$ | H | |
| 378 | Ethyl | H | H | H | O | $-CH_2-CH_2-CH_2-O-CH_2-$ | H | |
| 379 | $N(OCH_3)CH_3$ | H | H | H | O | $-CH(CH_3)-$ | 4-Phenyl | |
| 380 | $N(CH_3)_2$ | H | H | H | O | $-CH(CH_3)-$ | 4-Phenyl | |

Die Anilinderivate der Formel I können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken ; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate, z. B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z. B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz- Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenoläther, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen, Phospholipide und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind :

I. Man vermischt 90 Gewichtsteile der Verbindung Nr. 1 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 9 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

III. 20 Gewichtsteile der Verbindung Nr. 262 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

IV. 20 Gewichtsteile der Verbindung Nr. 1 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

V. 20 Gewichtsteile der Verbindung Nr. 27 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer

Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

VI. 5 Gewichtsteile der Verbindung Nr. 31 werden mit 95 Gewichtsteilen feinteiligem Kaolin vermischt. Man erhält auf diese Weise ein Stäubemittel, das 5 Gewichtsprozent des Wirkstoffs enthält.

VII. 30 Gewichtsteile der Verbindung Nr. 360 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile der Verbindung Nr. 31 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

Die Applikation der Wirkstoffe bzw. der Mittel kann im Vorauflaufverfahren oder im Nachauflaufverfahren erfolgen. Sind die Wirkstoffe für die Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Bekämpfungsziel und Wachstumsstadium 0,1 bis 15 kg/ha und mehr, vorzugsweise 0,5 bis 3 kg/ha.

Die herbizide Wirkung der Anilinderivate der Formel I läßt sich durch Gewächshausversuche zeigen :

Als Kulturgefäße dienen Plastikblumentöpfe mit 300 $cm^3$ Inhalt und lehmigem Sand mit etwa 1,5 % Humus als Substrat. Die Samen der Testpflanzen werden nach Arten getrennt flach eingesät. Unmittelbar danach erfolgt bei Vorauflaufbehandlung das Aufbringen der Wirkstoffe auf die Erdoberfläche. Sie werden hierzu in Wasser als Verteilungsmittel suspendiert oder emulgiert und mittels fein verteilender Düsen gespritzt. Bei dieser Applikationsmethode beträgt die Aufwandmenge 3,0 kg Wirkstoff/ha. Nach dem Aufbringen der Mittel beregnet man die Gefäße leicht, um Keimung und Wachstum in Gang zu bringen. Danach deckt man die Gefäße mit durchsichtigen Plastikhauben ab, bis die Pflanzen angewachsen sind. Diese Abdeckung bewirkt ein gleichmäßiges Keimen der Testpflanzen, sofern dies nicht durch die Wirkstoffe beeinträchtigt wird.

Zum Zwecke der Nachauflaufbehandlung zieht man die Testpflanzen je nach Wuchsform erst bis zu einer Wuchshöhe von 3 bis 15 cm an und behandelt sie danach. Hierzu werden entweder direkt gesäte und in den gleichen Gefäßen aufgewachsene Pflanzen ausgewählt, oder aber solche, die erst als Keimpflanzen getrennt angezogen und einige Tage vor der Behandlung in die Versuchsgefäße verpflanzt wurden. Zur Aufzucht der in den Nachauflaufversuchen werwendeten Sojabohnen setzt man der Topferde etwas Torf zu, um einen besseren Aufgang und ein günstigeres Wachstum zu erzielen. Die Aufwandmenge bei Nachauflaufanwendung beträgt 0,5 und 1,0 kg Wirkstoff/ha.

Die Versuchsgefäße werden im Gewächshaus aufgestellt, wobei für wärmeliebende Arten Bereiche von 20 bis 35 °C und für solche gemäßigter Klimate 10 bis 25 °C bevorzugt werden. Die Versuchsperiode erstreckt sich über 2 bis 4 Wochen. Während dieser Zeit werden die Pflanzen gepflegt, und ihre Reaktion auf die einzelnen Behandlungen wird ausgewertet. Bewertet wird nach einer Skala von 0 bis 100. Dabei bedeutet 100 kein Aufgang der Pflanzen bzw. völlige Zerstörung zumindest der oberirdischen Teile.

Die in den Gewächshausversuchen verwendeten Pflanzen setzen sich aus folgenden Arten zusammen :

Amaranthus spp. (Fuchsschwanz), Chenopodium album (Weißer Gänsefuß), Daucus carota (Möhren), Glycine max (Soja), Lolium multiflorum (Ital. Raygras), Mercurialis annua (Einjähriges Bingelkraut), Sesbania exaltata (Turibaum), Sinapis alba (Weißer Senf), Triticum aestivum (Weizen).

Bei der Prüfung auf herbizide Eigenschaften bekämpfen beispielsweise die Verbindungen Nr. 1, 27, 262 und 360 mit 3,0 kg Wirkstoff/ha unerwünschten Pflanzenwuchs bei Vorauflaufanwendung.

Bei Nachauflaufanwendung bekämpfen beispielsweise die Verbindung Nr. 31 mit 1,0 kg und die Verbindung Nr. 9 mit 0,5 kg Wirkstoff/ha unerwünschte Pflanzen und verhalten sich dabei selektiv in Kulturpflanzen.

In Anbetracht der Verträglichkeit und der Vielseitigkeit der Applikationsmethoden können die erfindungsgemäßen Verbindungen in einer großen Zahl von Kulturpflanzen zur Beseitigung unerwünschter Pflanzen eingesetzt werden.

In Betracht können beispielsweise folgende Kulturen kommen :

| Botanischer Name | Deutscher Name |
|---|---|
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |

| Botanischer Name | Deutscher Name |
|---|---|
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rübsen |
| Camellia sinensis | Teestrauch |
| Carthamus tinctorius | Saflor-Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cumumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum Gossypium herbaceum Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lens culinaris | Linse |
| Linum usitatissimum | Faserlein |
| Lycopersiocon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- u. Mehlbanane |
| Nicotiana tabacum (N. rustica) | Tabak |
| Olea europaea | Ölbaum |
| Oryza sativa | Reis |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Theobroma cacao | Kakaobaum |
| Trifolium pratense | Rotklee |
| Triticum aestivum | Weizen |

| Botanischer Name | Deutscher Name |
|---|---|
| Vaccinium carymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays (post directed) | Mais |

Zur Verbreiterung des Wirkungsspektrums und zur Erzielung synergistischer Effekte können die erfindungsgemäßen Verbindungen bzw. sie enthaltende herbizide Mittel mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Betracht.

Außerdem kann es von Nutzen sein, die Anilinderivate der Formel I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden. Es können auch nichtphytotoxische Öle und Ölkonzentrate zugesetzt werden.

## Patentansprüche

1. Anilinderivate der Formel

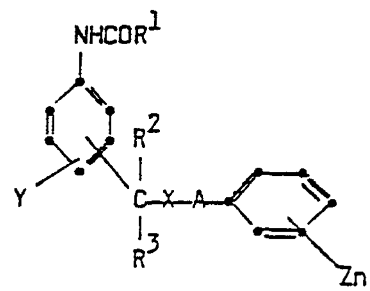

(I)

in der die Reste —$NHCOR^1$ und

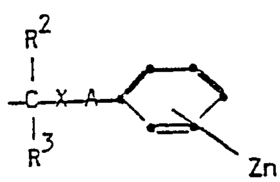

in m- oder p-Stellung stehen und

$R^1$ Alkoxy, Alkenoxy, Alkinoxy, Alkylthio oder gegebenenfalls durch Halogen oder Alkoxy mit 1 bis 5 Kohlenstoffatomen substituiertes Alkyl oder Alkenyl mit jeweils bis zu 4 Kohlenstoffatomen oder Cycloalkyl mit 3 bis 6 Kohlenstoffatomen oder den Rest —$NR^4R^5$, wobei $R^4$ und $R^5$ unabhängig voneinander Wasserstoff, Alkyl, Alkenyl, Alkinyl oder Alkoxy mit jeweils bis zu 4 Kohlenstoffatomen oder Cycloalkyl oder Cycloalkoxy mit 3 bis 6 Kohlenstoffatomen bedeuten oder $R^4$ und $R^5$ zusammen eine gegebenenfalls durch Methyl substituierte und gegebenenfalls Sauerstoff als Kettenglied enthaltende Alkylenkette mit bis zu 6 Kohlenstoffatomen bedeuten,

$R^2$ und $R^3$ unabhängig voneinander Wasserstoff oder Alkyl mit 1 bis 3 Kohlenstoffatomen,

X Sauerstoff oder den Rest —$NR^6$—, wobei $R^6$ ein Alkyl mit 1 bis 5 Kohlenstoffatomen bedeutet,

Y Wasserstoff, Halogen, Methyl, Methoxy oder Trifluormethyl,

A eine gegebenenfalls durch Alkyl mit 1 bis 5 Kohlenstoffatomen substituierte und gegebenenfalls Sauerstoff als Kettenglied enthaltende Alkylen- oder Alkenylenkette mit bis zu 5 Kohlenstoffatomen,

Z Wasserstoff, Halogen, Alkyl, Alkoxy, Halogenalkyl oder Halogenalkoxy mit jeweils bis zu 6 Kohlenstoffatomen, Phenyl oder gegebenenfalls durch Halogen, Nitro, Alkyl, Halogenalkyl oder Alkoxy mit jeweils 1 bis 3 Kohlenstoffatomen substituiertes Phenoxy und

n die Zahlen 1, 2 oder 3 bedeuten.

2. Anilinderivate der Formel I gemäß Anspruch 1, dadurch gekennzeichnet, daß $R^1$ den Rest $-N(OCH_3)(CH_3)$, $R^2$ Wasserstoff oder Methyl, $R^3$ Wasserstoff, X Sauerstoff, Y Wasserstoff oder Halogen, A Methylen oder Dimethylen, Z Halogen oder Alkyl mit 1 bis 4 Kohlenstoffatomen und n 0 oder 1 bedeuten.

3. Anilinderivate der Formel I gemäß Anspruch 1, dadurch gekennzeichnet, daß $R^1$ den Rest $-N(OCH_3)(CH_3)$, $R^2$ Wasserstoff oder Methyl, $R^3$ Wasserstoff, X Sauerstoff, Y Wasserstoff oder Halogen, A Methylen oder Dimethylen, Z Chlor, Methyl oder Methoxy und n 0 oder 1 bedeuten.

4. Verfahren zur Herstellung der Anilinderivate der Formel 1 gemäß Anspruch 1, dadurch gekennzeichnet, daß man ein Amin der Formel

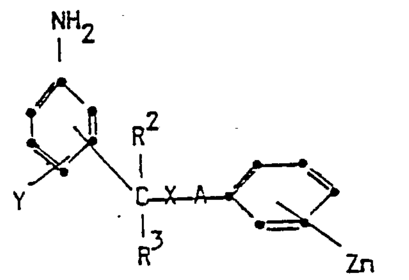

(II)

in der $R^2$, $R^3$, X, A, Y, Z und n die im Anspruch 1 genannten Bedeutungen haben, mit einer Verbindung der Formel

$$R^1\text{—CO—X} \qquad \text{(III)},$$

in der $R^1$ die im Anspruch 1 angegebenen Bedeutungen hat und X eine Abgangsgruppe oder den Rest $R^1$—CO—O— bedeutet, in Gegenwart eines inerten organischen Lösungsmittels und eines Säureakzeptors bei einer Temperatur im Bereich zwischen 20 und 80 °C umsetzt.

5. Verfahren zur Herstellung von Anilinderivaten der Formel I gemäß Anspruch 1, dadurch gekennzeichnet, daß man Verbindungen der Formel

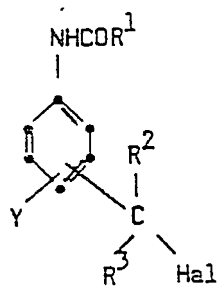

(IV)

in der Hal für Chlor oder Brom steht und $R^1$, $R^2$, $R^3$ und Y die im Anspruch 1 genannten Bedeutungen haben, mit einer Verbindung der Formel

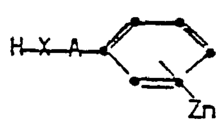

(V)

in der X, A, Z und n die im Anspruch 1 genannten Bedeutungen haben, gegebenenfalls in Gegenwart eines inerten Lösungsmittels oder in einem Überschuß der Verbindung der Formel V umsetzt.

6. Herbizid, enthaltend ein Anilinderivat der Formel I gemäß Anspruch 1.

7. Herbizid, enthaltend inerte Zusatzstoffe und ein Anilinderivat der Formel I gemäß Anspruch 1.

8. Herbizid, enthaltend inerte Zusatzstoffe und ein Anilinderivat der Formel I, in der $R^1$ den Rest $-N(OCH_3)(CH_3)$, $R^2$ Wasserstoff oder Methyl, $R^3$ Wasserstoff, X Sauerstoff, Y Wasserstoff oder Halogen, A Methylen oder Dimethylen, Z Halogen oder Alkyl mit 1 bis 4 Kohlenstoffatomen und n 0 oder 1 bedeuten.

9. Herbizid, enthaltend inerte Zusatzstoffe und ein Anilinderivat der Formel I, in der $R^1$ den Rest $-N(OCH_3)(CH_3)$, $R^2$ Wasserstoff oder Methyl, $R^3$ Wasserstoff, X Sauerstoff, Y Wasserstoff oder Halogen, A Methylen oder Dimethylen, Z Chlor, Methyl oder Methoxy und n 0 oder 1 bedeuten.

10. Verfahren zur Bekämpfung unerwünschten Pflanzenwuchses, dadurch gekennzeichnet, daß man eine herbizid wirksame Menge eines Anilinderivats der Formel I gemäß Anspruch 1 auf die Pflanzen und/oder die von ihnen freizuhaltende Fläche einwirken läßt.

**Claims**

1. An aniline derivative of the formula

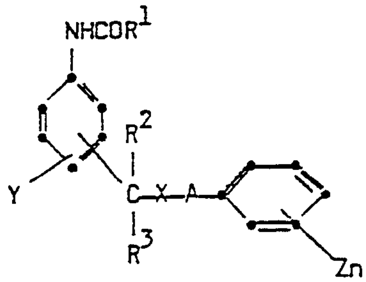

(I)

where the radicals $—NH—COR^1$ and

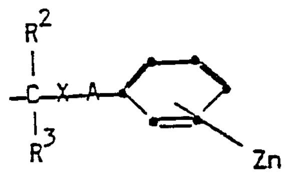

are meta or para to one another, and

$R^1$ is alkoxy, alkenoxy, alkynoxy or alkylthio, or is alkyl or alkenyl, each of which is of up to 4 carbon atoms and is unsubstituted or substituted by halogen or alkoxy of 1 to 5 carbons atoms, or is cycloalkyl of 3 to 6 carbon atoms or $—NR^4R^5$, where $R^4$ and $R^5$ independently of one another are hydrogen, alkyl, alkenyl, alkynyl or alkoxy, each of up to 4 carbon atoms, or cycloalkyl or cycloalkoxy of 3 to 6 carbon atoms, or $R^4$ and $R^5$ together form an unsubstituted or methylsubstituted alkylene chain of up to 6 carbon atoms which may or may not contain oxygen as a chain member,

$R^2$ and $R^3$ independently of one another are hydrogen or alkyl of 1 to 3 carbon atoms,

X is oxygen or $—NR^6—$, where $R^6$ is alkyl of 1 to 5 carbon atoms,

Y is hydrogen, halogen, methyl, methoxy or trifluoromethyl,

A is an alkylene or alkenylene chain of up to 5 carbon atoms which is unsubstituted or substituted by alkyl of 1 to 5 carbon atoms and may or may not contain oxygen as a chain member,

Z is hydrogen, halogen, alkyl, alkoxy, haloalkyl or haloalkoxy, each of up to 6 carbon atoms, or phenyl, or is phenoxy which is unsubstituted or substituted by halogen, nitro, alkyl, haloalkyl or alkoxy, each of 1 to 3 carbon atoms, and

n is 1, 2 or 3.

2. An aniline derivative of the formula I as claimed in claim 1, where $R^1$ is $—N(OCH_3)(CH_3)$, $R^2$ is hydrogen or methyl, $R^3$ is hydrogen, X is oxygen, Y is hydrogen or halogen, A is methylene or dimethylene, Z is halogen or alkyl of 1 to 4 carbon atoms, and n is 0 or 1.

3. An aniline derivative of the formula I as claimed in claim 1, where R is $—N(OCH_3)(CH_3)$, $R^2$ is hydrogen or methyl, $R^3$ is hydrogen, X is oxygen, Y is hydrogen or halogen, A is methylene or dimethylene, Z is chlorine, methyl or methoxy, and n is 0 or 1.

4. A process for the preparation of an aniline derivative of the formula I as claimed in claim 1, wherein an amine of the formula

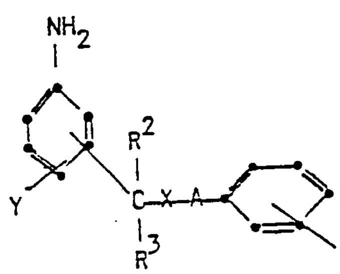

(II)

where $R^2$, $R^3$, X, A, Y, Z and n have the meanings given in claim 1, is reacted with a compound of the formula

$$R^1—CO—X \quad (III),$$

where $R^1$ has the meanings given in claim 1, and X is a leaving group or $R^1$—CO—O—, in the presence of an inert organic solvent and an acid acceptor at a temperature of from 20 to 80 °C.

5. A process for the preparation of an aniline derivative of the formula I as claimed in claim 1, wherein a compound of the formula

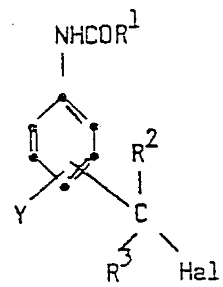

(IV)

where Hal is chlorine or bromine, and $R^1$, $R^2$, $R^3$ and Y have the meanings given in claim 1, is reacted with a compound of the formula

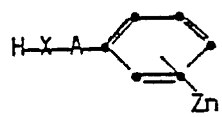

(V)

where X, A, Z and n have the meanings given in claim 1, either in the presence of an inert solvent or in an excess of the compound of the formula V.

6. A herbicide containing an aniline derivative of the formula I as claimed in claim 1.

7. A herbicide containing inert additives and an aniline derivative of the formula I as claimed in claim 1.

8. A herbicide containing inert additives and an aniline derivative of the formula I, where $R^1$ is —$N(OCH_3)(CH_3)$, $R^2$ is hydrogen or methyl, $R^3$ is hydrogen, X is oxygen, Y is hydrogen or halogen, A is methylene or dimethylene, Z is halogen or alkyl of 1 to 4 carbon atoms, and n is 0 or 1.

9. A herbicide containing inert additives and an aniline derivative of the formula I, where $R^1$ is —$N(OCH_3)(CH_3)$, $R^2$ is hydrogen or methyl, $R^3$ is hydrogen, X is oxygen, Y is hydrogen or halogen, A is methylene or dimethylene, Z is chlorine, methyl or methoxy, and n is 0 or 1.

10. A process for combating the growth of unwanted plants, wherein a herbicidally effective amount of an aniline derivative of the formula I as claimed in claim 1 is allowed to act on the plants and/or the area to be kept free of them.

**Revendications**

1. Dérivés d'aniline de la formule

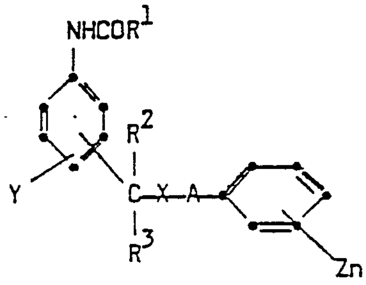

(I)

dans laquelle les restes —$NHCOR^1$ et

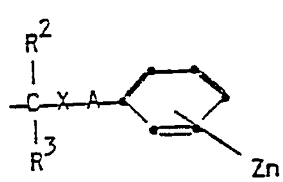

se trouvent en position m ou p, et

$R^1$ représente alcoxy, alcénoxy, alcinoxy, alkylthio ou alkyle ou alcényle, ayant chacun jusqu'à 4 atomes de carbone, éventuellement substitué par halogène ou alcoxy à 1 à 5 atomes de carbone, ou cycloalkyle ayant 3 à 6 atomes de carbone, ou le reste —$NR^4R^5$, $R^4$ et $R^5$, représentant, indépendamment l'un de l'autre, hydrogène, alkyle, alcényle, alcinyle ou alcoxy ayant chacun jusqu'à 4 atomes de carbone,

ou cycloalkyle ou cycloalcoxy ayant 3 à 6 atomes de carbone, ou $R^4$ et $R^5$ représentant ensemble une chaîne alkylène ayant jusqu'à 6 atomes de carbone éventuellement substituée par méthyle et contenant éventuellement de l'oxygène comme chaînon de la chaîne,

$R^2$ et $R^3$ représentent, indépendamment l'un de l'autre, hydrogène ou alkyle ayant 1 à 3 atomes de carbone,

X représente oxygène ou le reste —$NR^6$—, $R^6$ représentant un alkyle ayant 1 à 5 atomes de carbone,

Y hydrogène, halogène, méthyle, méthoxy ou trifluorométhyle,

A une chaîne alkylène ou alcénylène ayant jusqu'à 5 atomes de carbone, éventuellement substituée par alkyle ayant 1 à 5 atomes de carbone, et contenant éventuellement de l'oxygène comme chaînon de la chaîne,

Z hydrogène, halogène, alkyle, alcoxy, halogénalkyle ou halogénalcoxy ayant chacun jusqu'à 6 atomes de carbone, phényle ou phénoxy, éventuellement substitué par halogène, nitro, alkyle, halogénalkyle ou alcoxy, ayant chacun 1 à 3 atomes de carbone, et

n les nombres 1, 2 ou 3.

2. Dérivés d'aniline de formule I, selon la revendication 1, caractérisés par le fait que $R^1$ représente le reste —$N(OCH_3)(CH_3)$, $R^2$ représente hydrogène ou méthyle, $R^3$, hydrogène, X oxygène, Y hydrogène ou halogène, A méthylène ou diméthylène, Z halogène ou alkyle ayant 1 à 4 atomes de carbone et n représente 0 ou 1.

3. Dérivés d'aniline de formule I, selon la revendication 1, caractérisés par le fait que $R^1$ représente le reste —$N(OCH_3)(CH_3)$, $R^2$ représente hydrogène ou méthyle, $R^3$ hydrogène, X oxygène, Y hydrogène ou halogène, A méthylène ou diméthylène, Z chlore, méthyle ou méthoxy et n 0 ou 1.

4. Procédé de préparation de dérivés d'aniline de formule I, selon la revendication 1, caractérisé par le fait que l'on fait réagir, en présence d'un solvant inerte et d'un accepteur d'acide, à une température comprise entre 20 et 80 °C, une amine de formule

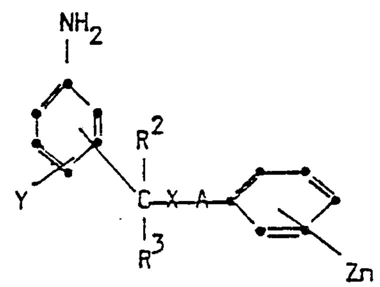

(II)

dans laquelle $R^2$, $R^3$, X, A, Y, Z et n ont les significations indiquées dans la revendications 1, avec un composé de formule

$$R^1\text{—CO—X} \qquad \text{(III)},$$

dans laquelle $R^1$ a la signification donnée dans la revendication 1 et X représente un groupe éliminable ou le reste $R^1$—CO—O—.

5. Procédé de préparation de dérivés d'aniline de formule I, selon la revendication 1, caractérisé par le fait que l'on fait réagir des composés de formule

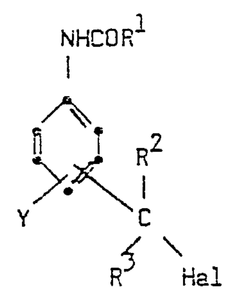

(IV)

dans laquelle Hal est mis pour chlore ou brome et $R^1$, $R^2$, $R^3$ et Y ont les significations données dans la revendication 1, avec un composé de formule

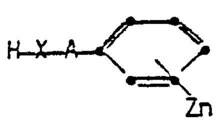

(V)

dans laquelle X, A, Z et n ont les significations données dans la revendication 1, éventuellement en présence d'un solvant inerte ou en un excès du composé de formule V.

6. Herbicide contenant un dérivé d'aniline de formule I, selon la revendication 1.

7. Herbicide contenant des additifs inertes et un dérivé d'aniline de formule I, selon la revendication 1.

8. Herbicide contenant des additifs inertes et un dérivé d'aniline de formule I, dans laquelle $R^1$ représente le reste $-N(OCH_3)(CH_3)$, $R^2$ représente hydrogène ou méthyle, $R^3$ hydrogène, X oxygène, Y hydrogène ou halogène, A methylène ou diméthylène, Z halogène ou alkyle ayant 1 à 4 atomes de carbone et n représente 0 ou 1.

9. Herbicide contenant des additifs inertes et un dérivé d'aniline de formule I, dans laquelle $R^1$ représente le reste $-N(OCH_3)(CH_3)$, $R^2$ représente hydrogène ou méthyle, $R^3$ hydrogène, X oxygène, Y hydrogène ou halogène, A méthylène ou diméthylène, Z chlore, méthyle ou méthoxy et n, 0 ou 1.

10. Procédé de lutte contre la croissance des plantes indésirables, caractérisé par le fait qu'on fait agir sur les plantes et/ou les surfaces à maintenir débarrassées de ces plantes, une quantité, efficace au point de vue herbicide, d'un dérivé d'aniline de formule I selon la revendication 1.